# EUROPÄISCHE PATENTANMELDUNG

(11) **EP 3 441 741 A1**
(43) Veröffentlichungstag der Anmeldung: **13.02.2019**
(21) Anmeldenummer: 17185845.9
(22) Anmeldetag: 11.08.2017
(51) Int. Cl.: G01N 1/34, B01D 61/24, B01D 61/28, B01D 63/08, G01N 30/14

(54) **DIALYSEZELLE ZUR PROBENVORBEREITUNG FÜR EIN CHEMISCHES ANALYSEVERFAHREN**

(71) Anmelder: METROHM AG, 9100 Herisau (CH)
(72) Erfinder: LÄUBLI, Markus, 9100 Herisau (CH); FRENZEL, Wolfgang, 10823 Berlin (DE); MARKEVICIUTE, Inga, 12353 Berlin (DE)
(74) Vertreter: Hepp Wenger Ryffel AG

(57) **Zusammenfassung**

Vorgeschlagen ist eine Dialysezelle (1) zur Probenvorbereitung für ein chemisches Analyseverfahren, insbesondere für die Ionenchromatographie. Die Dialysezelle (1) umfasst einen Donorkanal (2) und einen parallel dazu verlaufenden Akzeptorkanal (3). Der Donorkanal (2) und der Akzeptorkanal (3) sind durch eine selektivpermeable Dialysemembran (4) voneinander getrennt. Insbesondere ein in einer Donorlösung im Donorkanal (2) gelöster Analyt (5) kann durch die Dialysemembran (4) in die Akzeptorlösung im Akzeptorkanal (3) gelangen. Der Akzeptorkanal (3) hat zumindest abschnittsweise ein Volumen (*V_{A}*), das kleiner ist als ein parallel dazu verlaufendes Volumen (*V_{D}*) des Donorkanals (2).

## Beschreibung

Die vorliegende Erfindung betrifft eine Dialysezelle zur Probenvorbereitung für ein chemisches Analyseverfahren gemäss dem Oberbegriff von Anspruch 1, eine Verwendung einer derartigen Dialysezelle gemäss Anspruch 9 sowie eine Verwendung einer Membran als Dialysemembran in einer solchen Dialysezelle gemäss Anspruch 10.

Bei vielen chemischen Analyseverfahren ist es notwendig, eine Probe einer Vorbereitung zu unterziehen, bevor eine eigentliche Analyse durchgeführt werden kann. So enthalten beispielsweise Proben für die Analyse mittels Ionenchromatographie neben den zu analysierenden Ionen, dem oder den Analyten, häufig Matrixbestandteile, die korrosiv sind oder zu Ausfällungen führen können. Diese machen die Analyse mitunter schwierig oder gar unmöglich. Stark belastete Proben können darüber hinaus die Trennsäule beschädigen oder zu einer signifikanten Reduktion ihrer Lebensdauer führen. Zahlreiche Analytikanwendungen setzen eine geeignete Probenvorbereitung entsprechend zwingend voraus. Während traditionell sämtliche Probenvorbereitungsschritte manuell durchgeführt wurden, haben sich in den letzten Jahren sog. Inline-Probenvorbereitungstechniken durchgesetzt. Diese ermöglichen, das Vorbereitungsverfahren vollständig zu automatisieren.

So beschreibt die EP 0 820 804 A1 eine Inline-Dialyse, welche insbesondere für die Ionenchromatographie zur Anwendung kommt. Das besagte Dialyseverfahren wird mittels einer Dialysezelle durchgeführt, die einen Donorkanal sowie einen parallel dazu verlaufenden Akzeptorkanal aufweist. Zwischen diesen beiden Kanälen ist eine selektivpermeable Dialysemembran angeordnet. Die Dialyse wird als sog. Stopped-Flow-Dialyse durchgeführt. Dabei wird eine flüssige Probe, auch Donorlösung genannt, kontinuierlich durch den Donorkanal geleitet. Ein Flüssigkeitsstrom einer Akzeptorlösung durch den Akzeptorkanal wird zeitgleich für eine gewisse Dauer gestoppt. Durch den Konzentrationsgradienten zwischen der Donor- und der Akzeptorlösung kommt es zu einer Diffusion der Analyten von der Donorlösung in die Akzeptorlösung. Nachdem sich im Wesentlichen ein Konzentrationsgleichgewicht eingestellt hat, wird die Akzeptorlösung einer ionenchromatographischen Trennung zugeführt.

Die Zeitspanne vom Anhalten des Akzeptorstroms bis zum Ende der Dialyse, welches typischerweise durch ein Weiterleiten der Akzeptorlösung aus der Dialysezelle gegebenen ist, wird als Dialysezeit *t_{D}* bezeichnet.

Die Wiederfindungsrate R wird als Verhältnis der Analytenkonzentration im Akzeptorkanal zur Analytenkonzentration im Donorkanal zum Ende der Dialysezeit *t_{D}* definiert. Die Wiederfindungsrate *R* steigt typischerweise mit zunehmender Dialysezeit *t_{D}* streng monoton an.

Als Äquilibrationszeit *t_{A}* wird die Zeit bezeichnet, in der die Analytenkonzentration im Akzeptorkanal im Wesentlichen jene im Donorkanal erreicht hat. Im vorliegenden Zusammenhang entspricht die Äquilibrationszeit *t_{A}* derjenigen Dauer der Dialyse, nach welcher die relative Änderung der Analytenkonzentration im Akzeptorkanal erstmals weniger als 2%/min beträgt.

Die beschriebene Technik trennt nicht nur Partikel von dem oder den Analyten, sondern auch Kolloide, Ölbestandteile und grosse Moleküle. Insbesondere proteinhaltige Proben können damit direkt mittels Ionenchromatographie verarbeitet werden. Dies erspart zeitaufwändige manuelle Arbeitsschritte, wie beispielsweise eine Ausfällung der Proteine mittels Carrez-Reagens. Auch wenn Proben mit Partikeln belastet sind und eine Filtration aufgrund von blockierenden Filtern nicht angewendet werden kann, stellt die Inline-Dialyse eine praktikable Probenvorbereitung dar.

Obwohl diese Probenvorbereitungstechnik viele Vorteile hat, besteht in der Regel das Problem, dass die Dialyse der geschwindigkeitslimitierende Schritt des Analyseprozesses ist. Dies ist insbesondere bei einer seriellen Verarbeitung vieler Proben von Nachteil, da das Verfahren durch die Probenvorbereitung zeit- und kostenintensiv wird. Darüber hinaus besteht das Problem, dass es bei stark belasteten Proben zu einem teilweisen Durchbruch von Matrixverunreinigungen durch die Dialysemembran kommen kann. Ein weiterer Nachteil stellt der vergleichsweise hohe Probenaufwand bei der Stopped-Flow-Dialyse dar.

Es ist daher eine Aufgabe der vorliegenden Erfindung, die oben genannten Nachteile im Stand der Technik zu überwinden. Insbesondere ist es eine Aufgabe der vorliegenden Erfindung, die Äquilibrationszeit *t_{A}* zu verkürzen und damit einen insgesamt höheren Probendurchsatz zu erzielen. Darüber hinaus steht der vorliegenden Erfindung das Problem zu Grunde, bei stark belasteten Proben eine bessere Abtrennung des oder der Analyten von der Matrix zu erzielen.

Diese Aufgaben werden durch eine Dialysezelle zur Probenvorbereitung für ein chemisches Analyseverfahren, insbesondere für die Ionenchromatographie, gelöst, welche die Merkmale in Anspruch 1 aufweist. Die Dialysezelle umfasst einen Donorkanal und einen parallel dazu verlaufenden Akzeptorkanal. Der Donorkanal und der Akzeptorkanal sind bei bestimmungsgemässem Gebrauch durch eine selektivpermeable Dialysemembran voneinander getrennt. Dadurch kann insbesondere ein in einer Donorlösung im Donorkanal gelöster Analyt durch die Dialysemembran in die Akzeptorlösung im Akzeptorkanal gelangen. Der Akzeptorkanal hat zumindest abschnittsweise ein Volumen *V_{A}*, das kleiner ist als ein parallel dazu verlaufendes Volumen *V_{D}* des Donorkanals.

Es hat sich gezeigt, dass durch einen derartigen asymmetrischen Aufbau der Dialysezelle die Äquilibrationszeiten *t_{A}* markant gesenkt werden können. Dies führt zu kürzeren Dialysezeiten *t_{D}* und damit zu einem insgesamt höheren Probendurchsatz, was mit einer deutlichen Zeit- und Kostenersparnis verbunden ist. Darüber hinaus wird durch kürzere Dialysezeiten *t_{D}* der Zeitraum, während dem die Dialysemembran mit der Probenmatrix in Kontakt ist, reduziert. Dadurch kann der Durchbruch von Matrixverunreinigungen signifikant reduziert werden. Zudem kann die Dialyse mit einem deutlich geringeren Probenvolumen durchgeführt werden.

In einer bevorzugten Ausführungsform der vorliegenden Erfindung hat der Akzeptorkanal auf wenigstens 50%, vorzugsweise wenigstens 70%, bevorzugterweise wenigstens 90%, seiner Länge ein Volumen *V_{A}*, das kleiner ist als ein parallel dazu verlaufendes Volumen *V_{D}* des Donorkanals. In einer weiter bevorzugten Ausführungsform der vorliegenden Erfindung hat der Akzeptorkanal auf seiner gesamten Länge ein Volumen *V_{A}*, das kleiner ist als ein parallel dazu verlaufendes Volumen *V_{D}* des Donorkanals.

Der Akzeptorkanal kann zumindest abschnittsweise ein Volumen pro Längeneinheit *V_{A}*/*L* von 0.005 mm³/mm bis 2.0 mm³/mm, vorzugsweise von 0.020 mm³/mm bis 1.5 mm³/mm, bevorzugterweise von 0.10 mm³/mm bis 1.0 mm³/mm, haben. Der Donorkanal kann zumindest abschnittsweise ein Volumen pro Längeneinheit *V_{D}*/*L* von 0.25 mm³/mm bis 3.5 mm³/mm, vorzugsweise von 0.30 mm³/mm bis 3.0 mm³/mm, bevorzugterweise von 0.35 mm³/mm bis 2.0 mm³/mm, haben. Der Donor- und der Akzeptorkanal haben dabei üblicherweise je eine Länge von 10 mm bis 1000 mm, vorzugsweise von 20 mm bis 500 mm, bevorzugterweise von 100 mm bis 300 mm.

Es hat sich gezeigt, dass eine derartige Dimensionierung des Donor- und/oder des Akzeptorkanals insbesondere bei Anwendungen in der Ionenchromatographie vorteilhaft ist. Einerseits können damit deutlich kürzere Äquilibrationszeiten *t_{A}* erzielt werden. Andererseits ist mit diesen Dimensionen sichergestellt, dass die Stoffmenge an Analyt, die der Trennsäule zugeführt wird, innerhalb der Detektionsgrenzen eines üblichen Ionenchromatographiesystems liegt. Im Hinblick auf den Donorkanal ist mit diesen Abmessungen darüber hinaus sichergestellt, dass es auch bei stark, insbesondere mit Partikeln, belasteten Proben nicht zu einem Verstopfen desselben kommt.

Die selektivpermeable Dialysemembran kann eine Porengrösse von 0.01 µm bis 1.0 µm, vorzugsweise von 0.02 µm bis 0.5 µm, bevorzugterweise von 0.05 µm bis 0.2 µm, haben. Da durch den erfindungsgemässen asymmetrischen Aufbau der Dialysezelle mit derselben Dialysemembran eine erforderliche Wiederfindungsrate R schneller erreicht ist, kann für eine gegebene analytische Anwendung auch eine feinporigere Dialysemembran eingesetzt werden, ohne dass eine längere Äquilibrationszeit *t_{A}* in Kauf genommen werden müsste. Dadurch kann insbesondere bei stark belasteten Proben eine bessere Trennung der Matrix von dem oder den Analyten erzielt werden.

Die selektivpermeable Dialysemembran kann aus einem Material bestehen, welches ausgewählt ist aus einer Liste bestehend aus Zelluloseacetat, Zellulosenitrat, Polyvinylidenfluorid, Polycarbonat, Zellulosemischester, Zellulosehydrat und regenerierte Zellulose. Vorzugsweise ist das Material ausgewählt aus einer Liste bestehend aus Polyvinylidenfluorid, Polycarbonat und Zellulosemischester. Diese Membranmaterialien haben sich insbesondere bei der Trennung von Metall-Kationen und anorganischen Anionen als besonders vorteilhaft erwiesen.

Die Dialysezelle kann zwei Halbzellen umfassen, zwischen denen die selektivpermeable Dialysemembran angeordnet ist. Der Donorkanal und der Akzeptorkanal können als jeweils eine Vertiefung in einer Kontaktfläche einer der Halbzellen mit der Dialysemembran ausgebildet sein. Dieser Aufbau der Dialysezelle hat sich als besonders robust, kostengünstig in der Fertigung und benutzerfreundlich im Hinblick auf die Wartung erwiesen.

Der Querschnitt des Donorkanals und/oder des Akzeptorkanals kann kreissegmentförmig, insbesondere halbkreisförmig, halbellipsenförmig, quadratisch oder rechteckig sein. Diese Kanalgeometrien haben sich sowohl im Hinblick auf die Fertigung der Halbzellen als auch bezüglich ihrer Leistungscharakteristika als besonders vorteilhaft erwiesen.

Der Donorkanal und der Akzeptorkanal können linear, spiralförmig oder mäanderförmig ausgebildet sein. Dies ermöglicht in Relation zur Kanallänge eine besonders kompakte Ausgestaltung der Dialysezelle.

Die vorliegende Erfindung betrifft darüber hinaus die Verwendung einer Dialysezelle wie vorgängig beschrieben zur Probenvorbereitung in einem chemischen Analyseverfahren, insbesondere ausgewählt aus einer Liste bestehend aus Ionenchromatographie (IC), High Performance Liquid Chromatography (HPLC), Kapillarelektrophorese (CE) und Massenspektrometrie (MS).

Ein weiterer Aspekt der vorliegenden Erfindung betrifft die Verwendung einer Membran als Dialysemembran in einer Dialysezelle wie vorgängig beschrieben zur Probenvorbereitung für ein chemisches Analyseverfahren, insbesondere ausgewählt aus einer Liste bestehend aus Ionenchromatographie (IC), High Performance Liquid Chromatography (HPLC), Kapillarelektrophorese (CE) und Massenspektrometrie (MS).

Es versteht sich allerdings von selbst, dass im Rahmen der vorliegenden Erfindung auch Kopplungstechniken wie IC-MS, HPLC-MS oder CE-MS zur Anwendung kommen können.

Die beiden oben genannten Verwendungen können die folgenden Schritte umfassen:
- Bereitstellen einer Akzeptorlösung;
- Einleiten einer bestimmten Menge der Akzeptorlösung in den Akzeptorkanal oder in einen den Akzeptorkanal enthaltenden Akzeptorkreis;
- Halten der bestimmten Menge der Akzeptorlösung im Akzeptorkanal oder in den dem Akzeptorkanal enthaltenden Akzeptorkreis;
- Bereitstellen einer zumindest einen Analyten enthaltenden Donorlösung;
- Durchleiten der Donorlösung durch den Donorkanal, wodurch der zumindest eine in der Donorlösung enthaltene Analyt durch die Dialysemembran in die Akzeptorlösung gelangt.

Dabei wird mindestens solange frische Donorlösung durch den Donorkanal geleitet, bis die Konzentration des zumindest einen Analyten in der Akzeptorlösung mindestens 90%, vorzugsweise mindestens 95%, bevorzugterweise mindestens 99%, der Konzentration des zumindest einen Analyten in der Donorlösung beträgt.

Die oben genannte Verwendung kann zusätzlich das Zuführen der Akzeptorlösung in eine Analysevorrichtung, insbesondere ausgewählt aus einer Liste bestehend aus einer Ionenchromatographievorrichtung (IC), einer Vorrichtung für High Performance Liquid Chromatography (HPLC), einer Kapillarelektrophoresevorrichtung (CE) und einer Massenspektrometrievorrichtung (MS), umfassen.

Frische Donorlösung kann dabei kontinuierlich durch den Donorkanal geleitet werden. Die Flussrate der Donorlösung durch den Donorkanal kann in einem Bereich von 0.01 ml/min bis 10.0 ml/min, vorzugsweise von 0.05 ml/min bis 5.0 ml/min, bevorzugterweise von 0.10 ml/min bis 1.0 ml/min, liegen. Diese Flussraten haben sich im Hinblick auf die üblicherweise beobachtete Geschwindigkeit des Massenaustausches bei einer derartigen Dialyse bewährt.

Die vorliegende Erfindung betrifft darüber hinaus ein Analysesystem, insbesondere ein System ausgewählt aus einer Liste bestehend aus einem Ionenchromatographiesystem (IC), einem System für High Performance Liquid Chromatography (HPLC), einem Kapillarelektrophoresesystem (CE) und einem Massenspektrometriesystem (MS) oder einer Kombination solcher Systeme umfassend eine Dialysezelle wie oben beschrieben.

Weitere Vorteile und Einzelmerkmale der Erfindung ergeben sich aus der nachfolgenden Beschreibung eines Ausführungsbeispiels und aus den Zeichnungen.

Es zeigen schematisch:
- Figur 1:: Perspektivische Explosionsdarstellung einer erfindungsgemässen Dialysezelle;
- Figur 2:: Querschnittsdarstellung einer Dialysezelle aus dem Stand der Technik;
- Figur 3:: Querschnittsdarstellung einer erfindungsge-mässen Dialysezelle;
- Figur 4:: Ionenchromatographiesystem (IC) umfassend ei-ne erfindungsgemässe Dialysezelle;
- Figur 5:: Einfluss der Volumens *V_{A}* der Akzeptorkanals auf die Wiederfindungsrate *R*;
- Figuren 6 und 7:: Vergleich einer erfindungsgemässen Dialyse-zelle mit einer solchen aus dem Stand der Technik hinsichtlich des Durchbruchs von Lig-ninen.
- Figuren 8 und 9:: Beispiel einer Probenaufbereitung mit einer erfindungsgemässen Dialysezelle.

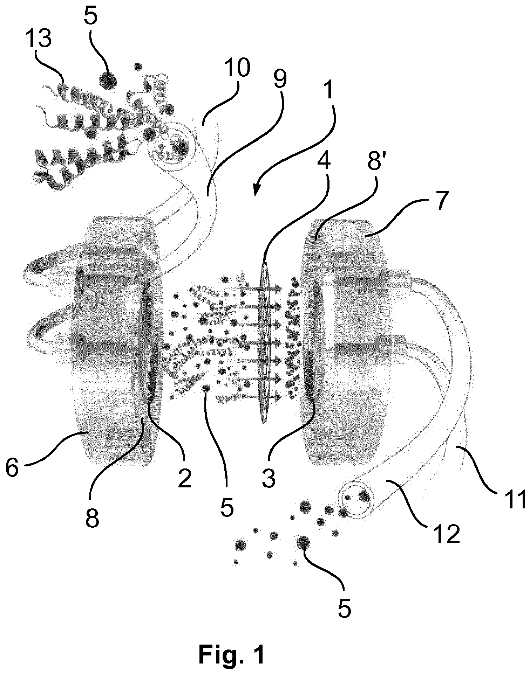

Wie aus Figur 1 ersichtlich ist, besteht eine Ausführungsform einer erfindungsgemässen Dialysezelle 1 aus zwei Halbzellen 6 und 7, zwischen denen eine selektivpermeable Dialysemembran 4 angeordnet ist. Die Halbzellen 6 und 7 weisen an ihren Kontaktflächen 8, 8' mit der Dialysemembran 4 jeweils eine spiralförmige Vertiefung auf, welche den Donorkanal 2 bzw. den Akzeptorkanal 3 bildet. Der Donorkanal 2 ist mit einer Zuleitung 9 verbunden, über welchen eine Probe zugeleitet werden kann. Darüber hinaus ist der Donorkanal 2 mit einer Ableitung 10 verbunden, mit welchem die Probe nach Durchlaufen der Dialysezelle 1 abgeleitet und üblicherweise einer Entsorgung 17 zugeführt werden kann. Auch der Akzeptorkanal 3 ist mit einer Zuleitung 11 und einer Ableitung 12 verbunden. Die Zuleitung 11 und die Ableitung 12 des Akzeptorkanals 3 können zu einem Akzeptorkreis zusammengeschlossen werden. Die durch den Donorkanal 2 geleitete Probenlösung enthält zumindest einen Analyten, der hier symbolisch als Metallion 5 dargestellt ist, sowie Matrixmoleküle, die vorliegend symbolisch als Proteine 13 dargestellt sind. Die Metallionen 5 können die selektivpermeable Dialysemembran 4 durchqueren (vgl. Pfeile), während die Proteine 13 zurückgehalten werden. Damit kann eine Metallionen 5 enthaltende Probe, beispielsweise für die Ionenchromatographie, aufbereitet werden.

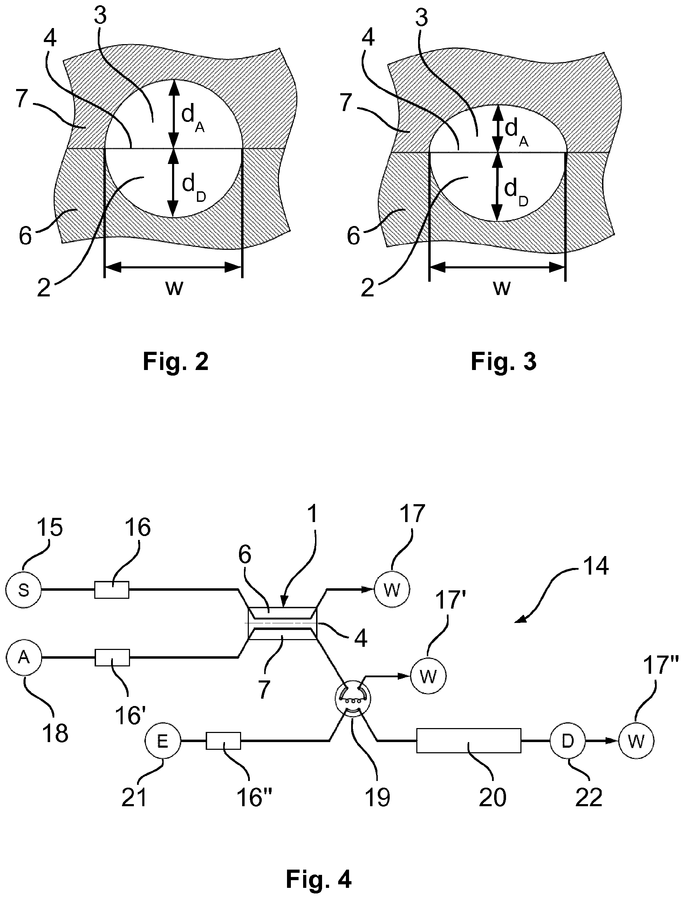

Die Figur 2 zeigt einen Querschnitt durch eine gattungsgemässe Dialysezelle aus dem Stand der Technik. Es ist zu erkennen, dass der Donorkanal 2 sowie der Akzeptorkanal 3 eine identische Breite w haben. Zudem ist auch die Tiefe *d_{D}* des Donorkanals gleich der Tiefe *d_{A}* des Akzeptorkanals. In der Figur 3 ist hingegen ein der Figur 2 entsprechender Querschnitt einer erfindungsgemässe Dialysezelle abgebildet. Sowohl der Donorkanal 2 als auch der Akzeptorkanal 2 weisen nach wie vor die Breite w auf. Auch ist die Tiefe des Donorkanals *d_{D}* gegenüber dem Beispiel gemäss Figur 2 identisch. Allerdings weist der Akzeptorkanal eine reduzierte Tiefe *d_{A}* auf. Es versteht sich von selbst, dass der Akzeptorkanal 3 damit im Bereich der Schnittebene ein Volumen pro Längeneinheit *V_{A}*/*L* aufweist, das kleiner ist als ein entsprechendes Volumen pro Längeneinheit *V_{D}*/*L* des Donorkanals 2.

Die Figur 4 zeigt ein Chromatographiesystem 14 umfassend eine erfindungsgemässe Dialysezelle 1. Im besagten System 14 ist eine Probenlösung, welche vorliegend auch als Donorlösung bezeichnet wird, in einem Probenbehälter 15 bereitgestellt. Die Donorlösung wird mittels einer Pumpe 16 durch die erste Halbzelle 6 der Dialysezelle 1 gepumpt und danach in einem Sammelbehälter 17 zwecks Entsorgung gesammelt. Eine Akzeptorlösung ist in einem Akzeptorbehälter 18 bereitgestellt und wird mittels einer weiteren Pumpe 16' durch die zweite Halbzelle 7 der Dialysezelle 1 gepumpt. Überschüssige Akzeptorlösung wird ebenfalls in einem Sammelbehälter 17' zwecks Entsorgung gesammelt. Wie vorgängig bereits erläutert wurde, wird zur Durchführung einer sogenannten Stopped-Flow-Dialyse der Strom der Akzeptorlösung, im Vorliegenden auch als Akzeptorstrom bezeichnet, durch die Halbzelle 7 gestoppt, während der Strom der Donorlösung, im Vorliegenden auch als Donorstrom bezeichnet, durch die Halbzelle 6 weitergeführt wird. Dies wird solange aufrechterhalten, bis die Akzeptorlösung innerhalb der Halbzelle 7 einen gewünschten Mindestanteil der Konzentration des Analyten der Donorlösung innerhalb der Halbzelle 6 aufweist. Ein solcher Mindestanteil kann beispielsweise 90%, 95% oder 99% sein.

Nach erfolgter Dialyse kann das Injektionsventil 19 umgeschaltet werden, wodurch der Analyt der Chromatographiesäule 20 zugeführt wird. Der eigentliche Chromatographieteil des Chromatographiesystems 14 ist vorliegend stark vereinfacht dargestellt. Ein Eluent ist in einem Eluentenbehälter 21 bereitgestellt und wird mittels einer Pumpe 16", insbesondere einer Hochdruckpumpe, über das Injektionsventil 19 durch die Trennsäule 20 gepumpt. Nach erfolgter Detektion mittels des Detektors 22 wird die ionenchromatographisch getrennte Probe ebenfalls in einem Sammelbehälter 17" zwecks Entsorgung gesammelt. Es versteht sich allerdings von selbst, dass im Rahmen der vorliegenden Erfindung auch sog. Tandemtechniken, beispielsweise eine Kopplung eines Leitfähigkeitsdetektors und eines Massenspektrometers (MS), realisierbar sind.

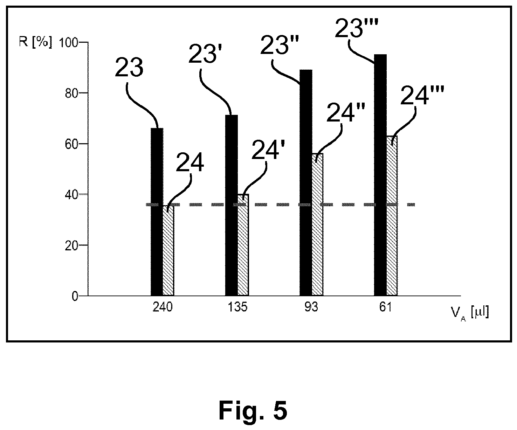

Die Figur 5 zeigt den Einfluss der Volumens *V_{A}* des Akzeptorkanals auf die Wiederfindungsrate R. Die Balken 23, 23', 23", 23"' stellen jeweils die beobachteten Werte für Chlorid dar, während die Balken 24, 24', 24", 24"' Daten für Sulfat repräsentieren.

Die dargestellten Werte zeigen jeweils die Wiederfindungsrate R nach 2 min Dialysezeit *t_{D}*. Für alle Messungen wurde ein Donorkanal mit einer Tiefe von 515 µm und einem Volumen *V_{D}* von 240 µl verwendet. Es wurden Akzeptorkanäle mit Volumina *V_{A}* von 240 µl, 135 µl, 93 µl und 61 µl getestet. Es ist zu erkennen, dass die Wiederfindungsrate R mit abnehmendem Volumen *V_{A}* des Akzeptorkanals bei gleichbleibender Dialysezeit *t_{D}* höher wird.

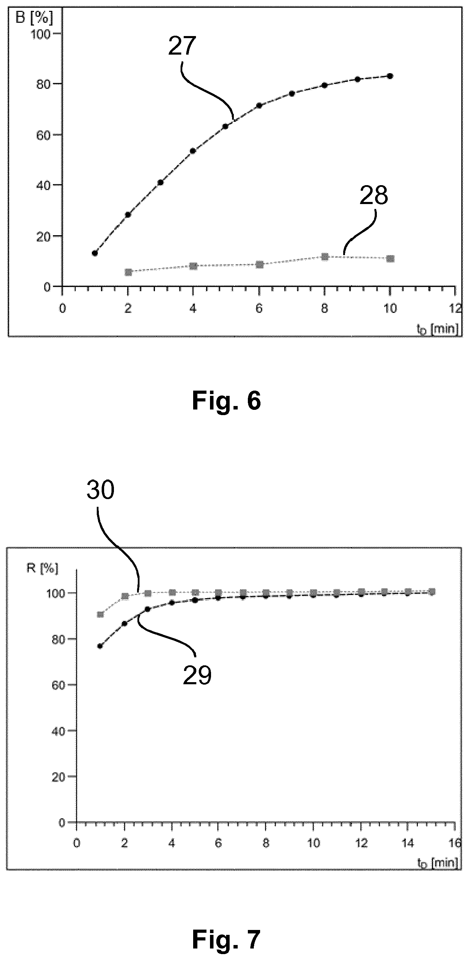

In den Figuren 6 und 7 ist ein Vergleich einer erfindungsgemässen Dialysezelle mit einer solchen aus dem Stand der Technik in Bezug auf den Durchbruch von Matrixmolekülen gezeigt. In diesem Zusammenhang wurde eine Probe mit einer Konzentration von Lignin von 25 mg/l hergestellt. Die Lignin-Konzentration wurde mit einem UV-Detektor bei einer Wellenlänge von 274 nm bestimmt. Der Graph 27 zeigt die Durchbruchsrate B von Lignin als Funktion der Dialysezeit *t_{D}* für eine symmetrische Dialysezelle mit einer Donor- bzw. Akzeptorkanaltiefe von 515 µm, einem Donor- bzw. Akzeptorkanalvolumen *V_{D}* bzw. *V_{A}* von 240µl und einer Zelluloseacetat-Membran mit einer Porengrösse von 0.2 µm. Es ist ersichtlich, dass die Durchbruchsrate *B* nach einer Dialysezeit *t_{D}* von 4 min über 70% liegt. Demgegenüber nimmt, wie in Graph 28 gezeigt, die Durchbruchsrate B für eine asymmetrische Dialysezelle mit einer Donorkanaltiefe von 515µm, einem Donorkanalvolumen *V_{D}* von 240µl, einer Akzeptorkanaltiefe von 515µm, einem Akzeptorkanalvolumen *V_{A}* von 90µl und einer Polycarbonat-Membran mit 0.1µm Porengrösse signifikant langsamer über die Dauer der Dialyse zu und erreicht selbst mit 10 min Dialysezeit *t_{D}* nur gerade einen Wert von 10%.

Die Figur 7 zeigt die Wiederfindungsrate R von Nitrat als Funktion der Dialysezeit *t_{D}* für die in Figur 6 gezeigten Dialysekonfigurationen. Graphen 29 und 30 zeigen die Wiederfindungsraten R von Nitrat entsprechend Graphen 27 bzw. 28 in Figur 6. Aus Figur 7 ist ersichtlich, dass der in Figur 6 gezeigte reduzierte Matrix-Durchbruch bei asymmetrischer Dialyse mit feinporiger Membran selbst für den Fall vorliegt, dass die Äquilibrationszeit *t_{A}* bei asymmetrischer Dialyse mit feinporiger Membran geringer ist als bei symmetrischer Dialyse mit grobporiger Membran.

Figuren 6 und 7 zeigen, dass durch die Verwendung einer asymmetrischen Dialysezelle gegenüber einer symmetrischen Dialysezelle durch die Wahl geeigneter Membranen die Äquilibrationszeit *t_{A}* für den Analyten verringert und gleichzeitig der Matrixdurchbruch verringert werden können.

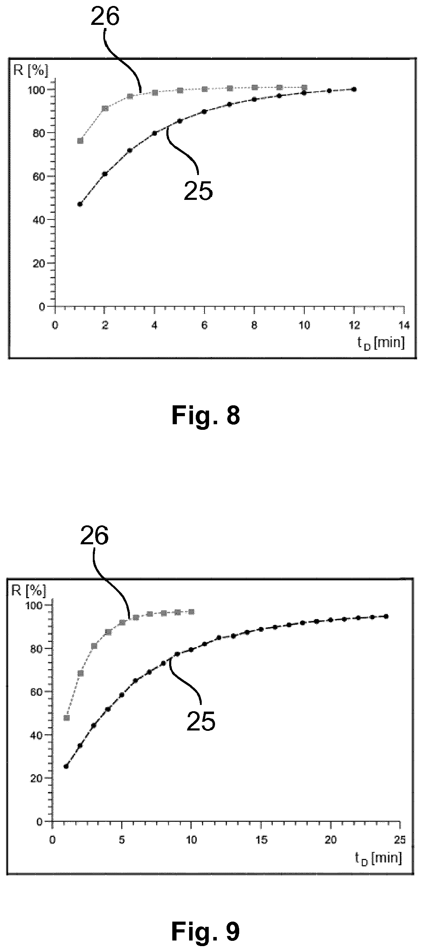

Nachfolgend sowie anhand der Figuren 8 und 9 ist ein Anwendungsbeispiel einer erfindungsgemässen Dialysezelle 1 gegeben. Namentlich wurde der Nitrat- und Sulfatgehalt von Wasserproben aus dem Britzer Kirchteich (Berlin, DE) ermittelt. Dabei handelt es sich um ein Oberflächengewässer, welches Zeichen von Eutrophierung sowie einen hohen Gehalt an Huminstoffen aufweist. Es wurden insgesamt fünf Wasserproben gesammelt und die Konzentration der besagten Anionen ionenchromatographisch bestimmt. Die Probenvorbereitung erfolgte mittels Stopped-Flow-Dialyse.

Es wurden zwei verschiedene Dialysezellen verwendet, welche jeweils einen Aufbau hatten, der dem in der Figur 1 gezeigten entspricht. Bei der ersten Dialysezelle handelte es sich um eine aus dem Stand der Technik bekannte, bei welcher sowohl der Donor- als auch der Akzeptorkanal eine Tiefe von 515 µm und ein Volumen *V_{A}* bzw. *V_{D}* von 240 µl aufwies. Bei der zweiten Dialysezelle handelte es sich um eine erfindungsgemässe, wobei der Donorkanal ebenfalls eine Tiefe von 515 µm und ein Volumen *V_{D}* von 240 µl aufwies. Der Akzeptorkanal hatte eine Tiefe von lediglich 210 µm und ein Volumen *V_{A}* von 90 µl.

In beiden Fällen wurde eine Membran aus Zellulosemischester mit einem Porendurchmesser von 0.05 µm (Merck Millipore) verwendet. Aufgrund der geringen Porengrösse zeichnet sich diese Membran durch eine starke Retention gegenüber potentiell störenden Substanzen, insbesondere makromolekularen Substanzen, wie beispielsweise Huminen oder Ligninen, aus.

Wie anhand der Figuren 8 und 9 gezeigt ist, wurde sowohl für Nitrat als auch für Sulfat erforderliche Dialysezeit *t_{D}* als Vorversuch bestimmt. Hierzu wurden für jedes Ion Standardlösungen mit einer Konzentration von 5 mg/l hergestellt und dialysiert. Die Figur 8 zeigt die Resultate für das Anion Nitrat. Der Graph 25 repräsentiert die Wiederfindungsrate R für einen Akzeptorkanal mit einem Volumen *V_{A}* von 240 µl. Der Graph 26 repräsentiert die entsprechende Wiederfindungsrate R bei einem Akzeptorkanal mit einem Volumen *V_{A}* von 90 µl. Es ist zu erkennen, dass bei der asymmetrischen Dialysezelle für Nitrat bereits mit einer Dialysezeit *t_{D}* von 2 min eine Wiederfindungsrate *R* von über 90% gegeben ist. Bei der symmetrischen Dialysezelle ist dieser Wert dagegen erst mit einer Dialysezeit *t_{D}* von über 6 min erzielbar. Die Figur 9 zeigt eine analoge Darstellung für das Anion Sulfat. Hier ist zu erkennen, dass eine Wiederfindungsrate R von 90% mit der asymmetrischen Dialysezelle mit ca. 5 min Dialysezeit *t_{D}* erreicht ist, während ein entsprechender Wert beim symmetrischen Zellenaufbau erst mit über 15 min Dialysezeit *t_{D}* erzielt werden kann. Die nachfolgende Tabelle fasst Resultate dieser Vorversuche für Nitrat und Sulfat sowie für weitere Anionen zusammen.

| **Anion** | **Dialysezeit *t_{D}* [min]** | **Wiederfindungsrate *R* [%]** | **Probenverbrauch [ml]** | **Dialysezeit *t_{D}* [min]** | **Wiederfindungsrate *R* [%]** | **Probenverbrauch [ml]** |
|---|---|---|---|---|---|---|
| | **240 µl Akzeptorkanal (515 µm Tiefe)** | | | **90 µl Akzeptorkanal (210 µm Tiefe)** | | |
| F⁻ | 15 | 97.2 | 11.6 | 6 | 98.3 | 5.4 |
| Cl⁻ | 9 | 97.1 | 7.5 | 4 | 99.0 | 4.1 |
| NO₂⁻ | 11 | 98.2 | 8.8 | 4 | 98.5 | 4.1 |
| Br⁻ | 9 | 98.4 | 7.5 | 3 | 98.3 | 3.4 |
| **NO₃⁻** | **9** | **97.1** | **7.5** | **3** | **98.8** | **3.4** |
| **SO₄²⁻** | **24** | **94.8** | **16.3** | **8** | **96.4** | **6.8** |

Wie der obigen Tabelle zu entnehmen ist, konnten mit der asymmetrischen Dialysezelle 1 für alle getesteten Anionen kürzere Dialysezeiten *t_{D}* erzielt werden. Die erreichten Wiederfindungsraten R lagen im gleichen Bereich wie bei einer symmetrischen Dialysezelle oder waren oft sogar höher. Darüber hinaus konnte mit einem asymmetrischen Aufbau der Dialysezelle grundsätzlich ein geringerer Probenverbrauch erzielt werden.

In der nachfolgenden Tabelle sind die Nitrat- und Sulfatgehalte für die oben genannte Oberflächenwasseranalyse zusammengefasst.

| **Probennummer** | **NO₃⁻** | | **SO₄²⁻** | |
|---|---|---|---|---|
| | **Konzentration [mg/l]** | **RSD [%]** | **Konzentration [mg/l]** | **RSD [%]** |
| 1 | 0.317 | 4.7 | 3.74 | 1.1 |
| 2 | 0.099 | 4.6 | 3.80 | 0.5 |
| 3 | 0.132 | 4.4 | 3.84 | 0.6 |
| 4 | 0.068 | 6.5 | 3.85 | 1.3 |
| 5 | 0.074 | 1.7 | 3.83 | 0.2 |

Neben dem Konzentrationswert für jede einzelne Probe ist darüber hinaus die relative Standardabweichung (RSD) der Konzentration angegeben.

Zusammenfassend ist festzuhalten, dass mit einer erfindungsgemässen Dialysezelle 1 mit asymmetrischem Aufbau deutlich kürzere Dialysezeiten *t_{D}* und damit ein höherer Probendurchsatz erzielt werden kann. Darüber hinaus wurde festgestellt, dass mit einer derartigen Dialysezelle 1 die Menge an benötigter Probe um mindestens einen Faktor 2 reduziert werden kann. Die geringere Zeit, während der die Matrix mit der Dialysemembran 4 in Kontakt ist, reduziert darüber hinaus den unerwünschten Durchbruch von Matrixbestandteilen sowie damit einhergehende negative Auswirkungen auf das Ionenchromatographiesystem.

## Patentansprüche

1. Dialysezelle (1) zur Probenvorbereitung für ein chemisches Analyseverfahren, insbesondere für die Ionenchromatographie, umfassend einen Donorkanal (2) und einen parallel dazu verlaufenden Akzeptorkanal (3), wobei der Donorkanal (2) und der Akzeptorkanal (3) bei bestimmungsgemässem Gebrauch durch eine selektivpermeable Dialysemembran (4) voneinander getrennt sind und insbesondere ein in einer Donorlösung im Donorkanal (2) gelöster Analyt (5) durch die Dialysemembran (4) in eine Akzeptorlösung im Akzeptorkanal (3) gelangen kann, **dadurch gekennzeichnet, dass** der Akzeptorkanal (3) zumindest abschnittsweise ein Volumen *V_{A}* hat, das kleiner ist als ein parallel dazu verlaufendes Volumen *V_{D}* des Donorkanals (2).

2. Dialysezelle (1) nach Anspruch 1, wobei der Akzeptorkanal (3) auf wenigstens 50%, vorzugsweise wenigstens 70%, bevorzugterweise wenigstens 90%, seiner Länge ein Volumen *V_{A}* hat, das kleiner ist als ein parallel dazu verlaufendes Volumen *V_{D}* des Donorkanals (2).

3. Dialysezelle (1) nach Anspruch 2, wobei der Akzeptorkanal (3) auf seiner gesamten Länge ein Volumen *V_{A}* hat, das kleiner ist als ein parallel dazu verlaufendes Volumen *V_{D}* des Donorkanals (2).

4. Dialysezelle (1) nach einem der Ansprüche 1 bis 3, wobei der Akzeptorkanal zumindest abschnittsweise ein Volumen pro Längeneinheit *V_{A}*/*L* von 0.005 mm³/mm bis 2.0 mm³/mm, vorzugsweise von 0.020 mm³/mm bis 1.5 mm³/mm, bevorzugterweise von 0.10 mm³/mm bis 1.0 mm³/mm, hat.

5. Dialysezelle (1) nach einem der Ansprüche 1 bis 4, wobei der Donorkanal (2) zumindest abschnittsweise ein Volumen pro Längeneinheit *V_{D}*/*L* von 0.25 mm³/mm bis 3.5 mm³/mm, vorzugsweise von 0.30 mm³/mm bis 3.0 mm³/mm, bevorzugterweise von 0.35 mm³/mm bis 2.0 mm³/mm, hat.

6. Dialysezelle (1) nach einem der Ansprüche 1 bis 5, wobei die Dialysemembran (4) eine Porengrösse von 0.01 µm bis 1.0 µm, vorzugsweise von 0.02 µm bis 0.5 µm, bevorzugterweise von 0.05 µm bis 0.2 µm, hat.

7. Dialysezelle (1) nach einem der Ansprüche 1 bis 6, wobei die Dialysemembran (4) aus einem Material besteht, ausgewählt aus einer Liste bestehend aus Zelluloseacetat, Zellulosenitrat, Polyvinylidenfluorid, Polycarbonat, Zellulosemischester, Zellulosehydrat und regenerierte Zellulose, vorzugsweise ausgewählt aus einer Liste bestehend aus Polyvinylidenfluorid, Polycarbonat und Zellulosemischester.

8. Dialysezelle (1) nach einem der Ansprüche 1 bis 7, umfassend zwei Halbzellen (6, 7), zwischen denen die Dialysemembran (4) angeordnet ist, wobei der Donorkanal (2) und der Akzeptorkanal (3) als jeweils eine Vertiefung in einer Kontaktfläche (8, 8') einer der Halbzellen (6, 7) mit der Dialysemembran (4) ausgebildet sind.

9. Dialysezelle (1) nach einem der Ansprüche 1 bis 8, wobei der Donorkanal (2) und der Akzeptorkanal (3) linear, spiralförmig oder mäanderförmig ausgebildet sind.

10. Verwendung einer Dialysezelle (1) nach einem der Ansprüche 1 bis 9 zur Probenvorbereitung in einem chemischen Analyseverfahren, insbesondere ausgewählt aus einer Liste bestehend aus Ionenchromatographie (IC), High Performance Liquid Chromatography (HPLC), Kapillarelektrophorese (CE) und Massenspektrometrie (MS).

11. Verwendung einer Membran als Dialysemembran (4) in einer Dialysezelle (1) nach einem der Ansprüche 1 bis 9 zur Probenvorbereitung für ein chemisches Analyseverfahren, insbesondere ausgewählt aus einer Liste bestehend aus Ionenchromatographie (IC), High Performance Liquid Chromatography (HPLC), Kapillarelektrophorese (CE) und Massenspektrometrie (MS).

12. Verwendung nach einem der Ansprüche 10 oder 11, umfassend die folgenden Schritte:
- Bereitstellen einer Akzeptorlösung;
- Einleiten einer bestimmten Menge der Akzeptorlösung in den Akzeptorkanal (3) oder in einen den Akzeptorkanal (3) enthaltenden Akzeptorkreis;
- Halten der bestimmten Menge der Akzeptorlösung im Akzeptorkanal (3) oder in dem den Akzeptorkanal (3) enthaltenden Akzeptorkreis;
- Bereitstellen einer zumindest einen Analyten (5) enthaltenden Donorlösung;
- Durchleiten der Donorlösung durch den Donorkanal (2), wodurch der zumindest eine in der Donorlösung enthaltene Analyt (5) durch die Dialysemembran (4) in die Akzeptorlösung gelangt,
wobei mindestens so lange frische Donorlösung durch den Donorkanal (2) geleitet wird, bis die Konzentration des zumindest einen Analyten (5) in der Akzeptorlösung mindestens 90%, vorzugsweise mindestens 95%, bevorzugterweise mindestens 99%, der Konzentration des zumindest einen Analyten (5) in der Donorlösung beträgt.

13. Verwendung nach Anspruch 12, zusätzlich umfassend den Schritt:
- Zuführen der Akzeptorlösung einer Analysevorrichtung, insbesondere ausgewählt aus einer Liste bestehend aus einer Ionenchromatographievorrichtung (IC), einer Vorrichtung für High Performance Liquid Chromatography (HPLC), einer Kapillarelektrophoresevorrichtung (CE) und einer Massenspektrometrievorrichtung (MS).

14. Verwendung nach einem der Ansprüche 12 oder 13, wobei die frische Donorlösung kontinuierlich durch den Donorkanal (2) geleitet wird.

15. Analysesystem, insbesondere ein Ionenchromatographiesystem (IC), einem System für High Performance Liquid Chromatography (HPLC), einem Kapillarelektrophoresesystem (EC) und einem Massenspektrometriesystem (MS), umfassend eine Dialysezelle (1) nach einem der Ansprüche 1 bis 9.
